# EUROPEAN PATENT SPECIFICATION

(11) **EP 1 304 115 B1**
(45) Date of publication and mention of the grant of the patent: **12.09.2007**
(21) Application number: 01945800.9
(22) Date of filing: 03.07.2001
(51) Int. Cl.: A61K 8/97, A61Q 99/00

(54) **ANTIPRURITIC COMPOSITIONS AND COMPOSITIONS PROMOTING WOUND HEALING**
ANTIPRURITISCHE ZUSAMMENSETZUNGEN UND DIE WUNDHEILUNG FÖRDERNDE ZUSAMMENSETZUNGEN
COMPOSITIONS ANTIPRURITIQUES ET COMPOSITIONS FAVORISANT LA CICATRISATION DES BLESSURES

(30) Priority: 26.07.2000 JP 2000224926; 26.07.2000 JP 2000224927
(43) Date of publication of application: 23.04.2003
(73) Proprietor: Hououdou Co. Ltd., Tokyo 142-0063 (JP)
(72) Inventor: TSUCHIDA, Yuuzou, Tokyo 142-0062 (JP); TSUCHIDA, Kotarou, Tokyo 142-0062 (JP); TSUCHIDA, Kenjirou, Tokyo 142-0062 (JP); TSURU, Sumiaki, Tokyo 203-0004 (JP)
(74) Representative: Müller-Boré & Partner Patentanwälte
(86) International application number: PCT/JP2001/005765
(87) International publication number: WO 2002/007745

(56) References cited:
- JP-A- 11 137 640
- JP-A- 11 196 818
- JP-A- 11 199 467
- JP-A- 11 199 502
- JP-A- 2000 128 731
- PATENT ABSTRACTS OF JAPAN vol. 008, no. 218 (C-245), 4 October 1984 (1984-10-04) & JP 59 104321 A (TEE OO SHII:KK), 16 June 1984 (1984-06-16)
- DATABASE BIOSIS [Online] BIOSCIENCES INFORMATION SERVICE, PHILADELPHIA, PA, US; 1982 VAN CHUYEN N ET AL: "ANTI MICROBIAL ACTIVITY OF KUMAZASA SASA-ALBO-MARGINATA" Database accession no. PREV198375008762 XP002279698 & AGRICULTURAL AND BIOLOGICAL CHEMISTRY, vol. 46, no. 4, 1982, pages 971-978, ISSN: 0002-1369
- PATENT ABSTRACTS OF JAPAN vol. 2000, no. 05, 14 September 2000 (2000-09-14) & JP 2000 044481 A (SUNSTAR INC), 15 February 2000 (2000-02-15)

## Description

### Technical Field

The present invention relates to an antipruritic composition and a wound-healing-promoting composition, which comprise an extract from Sasa albo-marginata (Bambooseae Sasa) as an effective component.

### Prior Art

Recently, there have rapidly been increased the number of patients suffering from inflammatory skin diseases accompanied by itching such as systemic itching derived from atopic dermatitis, senile itch, diabetes and hepatitis C; itching derived from vulval itch observed during the menopause and climacterium; itching originated from fungi such as itching derived from candidosis, tinea cruris, athlete's foot and tinea; itching observed when patients are in the convalescent stage of miliaria, tumors, rashes, bedsore, insect bites, burns and cuts; and itching due to drug-induced injuries such as sore originated from liquids for hairdressing and beautification such as permanent wave lotion solutions and hair-coloring solutions (hereunder also referred to as "atopy or the kike") and this becomes a serious problem. In this respect, it has been recognized that this is caused since the itching and pains are feeling accompanied by the inflammation and the consciousness induced when one is inflamed resides in a wide range extending from pains to itching.

In most cases, a steroidal drug has been used for alleviating and treating itching and pains. When a steroidal drug is administered to babies and little children, however, such symptoms are often worsened and infectious diseases get worse since the steroidal drug would reduce the immunity of the infants. Moreover, the appearance of cases in which any conventional steroidal drug is not effective makes the problems such as the atopy more serious.

Accordingly, naturally occurring vegetable components have attracted special interest recently as substitutes for the conventional steroidal drugs. For instance, it has been reported that Sasa albo-marginata has an anti-allergic activity since early times. The inventors of this invention have likewise found that an extract from Sasa albo-marginata would significantly increase, in mice, the anti-inflammatory effect, antibiotic action and wound-healing-promoting action.

However, conventional compositions containing Sasa albo-marginata extract have almost no effect of improving the symptoms of, for instance, atopy and no antipruritic action and are insufficient in its wound-healing-promoting effect.

For this reason, there has been desired for the development of an antipruritic composition and a wound-healing-promoting composition, which are excellent in the effect of alleviating symptoms of, for instance, atopy and in the antipruritic effect and which can be used for treating or alleviating the itching associated with the skin diseases including atopy.

### Summary of the Invention

Accordingly, it is an object of the present invention to provide an antipruritic composition having a high antipruritic action against skin diseases and skin disorders such as atopy and more particularly the inflammatory skin diseases and skin disorders accompanied by itching, for instance, systemic itching derived from atopic dermatitis, senile itch, diabetes and hepatitis C; itching derived from vulval itch observed during the menopause and climacterium; itching originated from fungi such as itching derived from candidosis, tinea cruris, athlete's foot and tinea; itching observed when patients are in the convalescent stage of miliaria, tumors, rashes, bedsore, insect bites, burns and cuts; and itching due to drug-induced injuries such as sore originated from liquids for hairdressing and beautification such as permanent wave lotion solutions and hair-coloring solutions.

It is another object of the present invention to provide a wound-healing- promoting composition having a high wound-healing-promoting effect against the foregoing inflammatory skin diseases and skin disorders.

Accordingly, the present invention herein provides an antipruritic composition, which comprises a water extract from Sasa albo-marginata (Bambooseae Sasa) in an amount ranging from 1 to 10% by mass as expressed in terms of the solid content of the extract.

The present invention also provides the use of a composition, which comprises an extract from Sasa albo-marginata (Bambooseae Sasa) in an amount ranging from 1 to 10% by mass as expressed in terms of the solid content of the extract, for promoting wound-healing.

### Best Mode for Carrying Out the Invention

The present invention thus relates to an antipruritic composition, which comprises, as an effective component, a water extract from Sasa albo-marginata (Bambooseae Sasa) in an amount ranging from 1 to 10% by mass, preferably 2 to 8% by mass and more preferably 3 to 6% by mass as expressed in terms of the solid content of the extract as well as a wound-healing-promoting composition, which comprises an extract from Sasa albo-marginata (Bambooseae Sasa) in an amount ranging from 1 to 10% by mass, preferably 2 to 8% by mass and more preferably 3 to 6% by mass as expressed in terms of the solid content of the extract. If the solid content of the extract in the compositions is less than 1% by mass, the resulting compositions are insufficient in their intended antipruritic and wound-healing-promoting effects, while if the solid content exceeds 10% by mass, the resulting composition have stronger irritating actions for the skin.

Conventionally, the Sasa albo-marginata extract has been prepared in the form of an extract having a solid content ranging from 0.5 to 10% by mass and has been used in a variety of applications. The extract having such a solid content has been used in such a manner that the content thereof in a final product in general ranges from 1 to 10% by mass and therefore, the solid content of the Sasa albo-marginata extract in the final product in general ranges from about 0.05 to 0.8% by mass and at highest on the order of less than 1% by mass. The reason for this is, for instance, that the Sasa albo-marginata extract is relatively expensive, that the extract would show the anti-inflammatory effect and antibiotic action even in such a low concentration to some extent and that it would be unreasonable to increase the added amount of the effective component to a level of not less than 10% by mass. However, the product having such a low content of the extract shows almost no effect of ameliorating, for instance, atopy and antipruritic effect and it is also insufficient in the wound-healing-promoting effect.

The inventors of this invention have found that if the Sasa albo-marginata extract is present in a final product in a solid content ranging from 1 to 10% by mass, preferably 2 to 8% by mass and more preferably 3 to 6% by mass, the product shows a highly improved effect of ameliorating symptoms of, for instance, atopy and a highly improved antipruritic effect, which have never been recognized when using a conventional extract having a low solid content and that the former also shows a considerably improved wound-healing-promoting effect and have thus completed the present invention. The Sasa albo-marginata extract per se has long been known, but there has never been tried any attempt to use an extract in such a high solid content. The reason for this has not yet been elucidated, but it has surprisingly been recognized that the use of such an extract in a concentration higher than that of the conventionally used one (not less than 10 times) would permit the significant improvement of the effect of ameliorating the symptoms of, for instance, atopy and the antipruritic effect and would also permit the considerable improvement of the wound-healing-promoting effect.

The Sasa albo-marginata used in the present invention, as a raw material for the extract thereof is not restricted to any specific one, but preferably used herein is one collected in, for instance, TESHIO Mountains in Hokkaido during the term extending from July to October.

The Sasa albo-marginata extract used in the present invention is preferably one prepared by extracting raw leaves or dried leaves of Sasa albo-marginata, preferably dried leaves thereof with water maintained at a temperature ranging from 60 to 130°C at ordinary pressure or while applying a pressure.

The extraction method is not restricted to any particular one, but usable herein includes, for instance, that disclosed in Japanese UnExamined Patent Publication No. Hei 11-196818. More specifically, leaves of Sasa albo-marginata are extracted at a temperature ranging from 110 to 130°C for 5 to 30 minutes using a pressurized hot water extraction device, the resulting extract is separated into a moisture-containing solid content (moisture content: 40 to 70%) in a moisture separator, thereafter the moisture-containing solid content is treated at a temperature ranging from 130 to 200°C for 5 to 30 minutes in a saturated vapor-heating device, the solid content thus treated is again treated at 110 to 130°C for 5 to 30 minutes using a pressurized hot water extraction device and the extracts obtained in the first and second extraction steps are combined prior to practical use. Alternatively, it is also possible to use an extract obtained by extracting dried leaves of Sasa albo-marginata with, for instance, water heated to 60 to 100°C for 30 minutes to 12 hours.

The Sasa albo-marginata extract thus obtained contains sulfur-containing components and the content thereof as expressed in terms of the amount of sulfur ranges from about 4 to 10 mg and usually about 6 to 9 mg per one gram of the solid content of the Sasa albo-marginata extract. Principal constituents of the sulfur- containing components are considered to be sulfur-containing amino acids.

The antipruritic and wound-healing-promoting compositions of the present invention comprise such sulfur-containing components derived from the Sasa albo-marginata extract in an amount preferably ranging from 4 to 100 mg, more preferably 8 to 80 mg and most preferably 12 to 60 mg per 100 g of each composition as expressed in terms of the amount of sulfur.

In the preparation of the antipruritic composition and the wound-healing- promoting composition of the present invention, there may be used, for instance, a basic component such as an oily component, a moisture retentive agent and/or a preservative, which are commonly used in pharmaceutical compositions, cosmetics and compositions applied to the skin in addition to a desired amount of the foregoing Sasa albo-marginata extract.

The origins of the water used in the composition are not restricted to particular ones and examples thereof include tap water, natural water and purified water, but preferably used herein is highly purified water such as ion-exchange water.

Examples of oily components usable herein are oils derived from animals such as squalane, tallow, lard, horse fat, lanolin and beeswax; oils derived from vegetables such as olive oil, grape seed oil, palm oil, jojoba oil and germ oil (such as rice germ oil); and synthetic or semi-synthetic oils such as liquid paraffin, higher fatty acid esters (such as octyl palmitate, isopropyl palmitate and octyl dodecyl myristate) and silicone oil.

The oily components are used in appropriate combinations depending on the performance requirement, for instance, an ability of protecting the skin, an effect of imparting emollient (or an effect of preventing drying of the skin and imparting softness and resilience to the skin through the coverage of the skin surface with a thin film) and an ability of imparting refreshed feeling to the skin. In one of preferred examples of such combinations, the oily component comprises squalane, olive oil and octyl dodecyl myristate.

The composition comprises a solid oil component such as stearic acid, stearyl alcohol, behenic acid, cetanol and vaseline to control the hardness and flowability of the composition and the composition preferably comprises stearic acid and cetanol in combination.

When preparing the antipruritic composition and the wound-healing-promoting composition of the present invention in the form of a cream composition, a creaming agent is used to convert the mixture of Sasa albo-marginata extract, water and an oily component into a cream. Such a creaming agent is not restricted to any particular one, but glycerin monostearate and a self-emulsifiable glycerin monostearate (a product obtained by incorporating an emulsifying agent into glycerin monostearate) are in general used in combination.

Moreover, the antipruritic composition and the wound-healing-promoting composition of the present invention may, if necessary, contain other additives such as a stabilizer, a moisture retentive agent, a wound-healing agent, a preservative and/or a surfactant.

Examples of stabilizers are a combination of a carboxy vinyl polymer with potassium hydroxide, and polyethylene glycol distearate. In particular, polyethylene glycol sesqui-stearate (a 1:1 mixture of polyethylene glycol distearate and polyethylene glycol monostearate) (molecular weight of the polyethylene glycol ranging from 1000 to 20,000) is preferably used herein since it has high stability, it is never separated into water and oil and the hardness required when the composition is applied to the skin in the form of a cream composition can effectively be controlled.

Examples of moisture retentive agents usable herein are sodium salt of hyaluronic acid, collagen, aloe extract (in particular, aloe extract (2) derived from Aloe arborescens is preferred), urea, 1,3-butylene glycol, glycerin, trehalose, sorbitol, amino acids and sodium salt of pyrrolidone carboxylic acid.

Examples of wound-healing agents usable herein are allantoin, dipotassium glycyrrhizinate, glycyrrhiza extract and mugwort extract.

The preservative is used subsidiarily since the Sasa albo-marginata extract has an antibiotic effect by nature. Examples of such preservatives are sodium benzoate, lower alkyl esters of p-hydroxy benzoic acid (for instance, so-called paraben such as methyl, ethyl, propyl or butyl ester), sodium propionate, mixed fatty acid esters (a mixture of capric acid glyceryl, lauric acid polyglyceryl-2 and lauric acid polyglyceryl-10), phenoxytal, and light-sensitive substance No. 201 (yellow dye), with paraben and mixed fatty acid esters being preferably used herein.

Examples of surfactants are sodium N-acyl-L-glutamate and polyoxyethylene sorbitan monostearate.

In addition, the composition may, if required, comprise aroma components such as orange oil, lemon oil, bitter orange peel oil and perfumes.

Water is added to the foregoing components to make the total amount of the resulting composition 100% by mass.

The following Table 1 shows preferred amounts (% by mass) of the foregoing ingredients required for preparing the antipruritic composition and the wound- healing-promoting composition in the form of a cream composition. The amount of each component other than water corresponds to the mass thereof free of any moisture.

**Table 1**

| Component | Preferred range | More preferred range | Most preferred range |
|---|---|---|---|
| Sasa albo-marginata extract | 1 to 10 | 2 to 8 | 3 to 6 |
| Liquid oily component | 6 to 30 | 2 to 20 | 5 to 15 |
| Solid oily component | 2 to 35 | 3 to 25 | 5 to 15 |
| Creaming agent | 1 to 6 | 1.5 to 4 | 1.6 to 3 |
| Stabilizer | 0 to 2 | 0 to 1.5 | 0 to 1 |
| Moisture retentive agent | 0 to 10 | 0.05 to 5 | 0.1 to 5 |
| Wound-healing agent | 0 to 2 | 0.05 to 1 | 0.1 to 0.5 |
| Aroma component | 0 to 5 | 0 to 3 | 0 to 1 |
| Water | Balance | Balance | Balance |

The foregoing components are introduced into a heating-mixing kettle equipped with a stirring blade and preferably an emulsification apparatus and they are then admixed together at 70 to 90°C for one to two hours to form an antipruritic composition or wound-healing-promoting composition of the present invention.

The antipruritic composition or wound-healing-promoting composition of the present invention may be used in the form of shapes other than a cream composition such as an ointment, a liquid and a jelly with a cream composition being preferred because it can easily be used and shows a considerably effect. Moreover, the composition of the present invention may likewise be formed into a solid or liquid product such as shampoo, body soap and soap.

It is sufficient to apply the antipruritic composition or wound-healing-promoting composition of the present invention, after cleaning the affected part, an appropriate amount thereof, for instance, 0.1 to 1 g per 100 cm² of the skin in case of a cream composition 1 to 5 times, usually 1 to 3 times a day. The amount and number of application of the composition may appropriately be changed while taking into consideration, for instance, the symptoms of atopy and the extent of a particular wound. The antipruritic composition or wound-healing-promoting composition of the present invention would in general permit the alleviation or elimination of itching immediately after the application thereof to the affected part.

The antipruritic composition or wound-healing-promoting composition of the present invention comprises 1 to 10% by mass of the Sasa albo-marginata extract as expressed in terms of the solid content and accordingly, the composition would show considerable effects of improving the symptoms of atopy to which any conventional composition has almost no effect and of promoting the wound-healing.

The following are preferred embodiments of the composition according to the present invention.
1. An antipruritic or wound-healing-promoting composition which comprises the Sasa albo-marginata extract (1 to 10% by mass as expressed in terms of the solid content), water, an oily component and a creaming agent.
2. The composition according to the foregoing item 1 in which the oily component is at least one member selected from the group consisting of animal oil, vegetable oil, synthetic oil and semi-synthetic oil.
3. The composition according to the foregoing item 1 in which the oily component is at least one member selected from the group consisting of squalane, tallow, lard, horse fat, lanolin, beeswax, olive oil, grape seed oil, palm oil, jojoba oil, germ oil, liquid paraffin, octyl palmitate, isopropyl palmitate, octyl dodecyl myristate, silicone oil, stearic acid, stearyl alcohol, behenic acid, cetanol and vaseline.
4. The composition according to any one of the foregoing items 1 to 3 in which the creaming agent is a combination of glycerin monostearate with self-emulsifiable glycerin monostearate.
5. The composition according to any one of the foregoing items 1 to 4 which further comprises at least one component selected from the group consisting of a stabilizer, a moisture retentive agent, a wound-healing agent, a preservative and a surfactant.
6. The composition according to the foregoing item 5 in which the stabilizer is at least one member selected from the group consisting of a combination of a carboxy vinyl polymer with potassium hydroxide, and polyethylene glycol distearate.
7. The composition according to the foregoing item 5 in which the moisture retentive agent is at least one member selected from the group consisting of sodium salt of hyaluronic acid, collagen, aloe extract, urea, 1,3-butylene glycol, glycerin, trehalose, sorbitol, amino acids and sodium salt of pyrrolidone carboxylic acid.
8. The composition according to the foregoing item 5 in which the wound-healing agent is at least one member selected from the group consisting of allantoin, dipotassium glycyrrhizinate, glycyrrhiza extract and mugwort extract.
9. The composition according to the foregoing item 5 in which the preservative is at least one member selected from the group consisting of sodium benzoate, lower alkyl esters of p-hydroxy benzoic acid, sodium propionate, mixed fatty acid esters, phenoxytal, and yellow dye.
10. The composition according to the foregoing item 5 which further comprises at least one member selected from the group consisting of orange oil, lemon oil, bitter orange peel oil and perfumes.
11. The composition according to the foregoing item 10, which comprises a Sasa albo-marginata extract, water, an oily component, a creaming agent, a stabilizer, a moisture retentive agent, a wound-healing-promoting agent, a preservative and a surfactant, wherein the oily component is at least one member selected from the group consisting of squalane, tallow, lard, horse fat, lanolin, beeswax, olive oil, grape seed oil, palm oil, jojoba oil, germ oil, liquid paraffin, octyl palmitate, isopropyl palmitate, octyl dodecyl myristate, silicone oil, stearic acid, stearyl alcohol, behenic acid, cetanol and vaseline; the creaming agent is a combination of glycerin monostearate with self-emulsifiable glycerin monostearate; the stabilizer is at least one member selected from the group consisting of a combination of a carboxy vinyl polymer with potassium hydroxide and polyethylene glycol distearate; the moisture retentive agent is at least one member selected from the group consisting of sodium salt of hyaluronic acid, collagen, aloe extract, urea, 1,3-butylene glycol, glycerin, trehalose, sorbitol, amino acids and sodium salt of pyrrolidone carboxylic acid; the wound-healing agent is at least one member selected from the group consisting of allantoin, dipotassium glycyrrhizinate, glycyrrhiza extract and mugwort extract; the preservative is at least one member selected from the group consisting of sodium benzoate, lower alkyl esters of p-hydroxy benzoic acid, sodium propionate, mixed fatty acid esters, phenoxytal, and yellow dye; and the surfactant is sodium N-acyl-L-glutamate.
12. The composition as set forth in the foregoing item 11 which further comprises at least one member selected from the group consisting of orange oil, lemon oil, bitter orange peel oil and perfumes.
13. The composition as set forth in the foregoing item 1 which comprises a Sasa albo-marginata extract, water, squalane, olive oil, glycerin monostearate, self- emulsifiable glycerin monostearate, a carboxy vinyl polymer, potassium hydroxide, urea, 1,3-butylene glycol, allantoin, a lower alkyl ester of p-hydroxy benzoic acid, stearic acid, sodium N-acyl-L-glutamate and lemon oil.
14. The composition as set forth in the foregoing item 1 which comprises a Sasa albo-marginata extract, water, squalane, olive oil, octyl dodecyl myristate, cetanol, glycerin monostearate, self-emulsifiable glycerin monostearate, a carboxy vinyl polymer, potassium hydroxide, urea, 1,3-butylene glycol, allantoin, a mixed fatty acid ester, stearic acid, sodium N-acyl-L-glutamate and orange oil.
15. The composition as set forth in any one of the foregoing items 1 to 14 which comprises polyethylene glycol sesqui-stearate.

The present invention will hereunder be described in more detail with reference to the following Reference Examples, working Examples and Test Examples.

### Reference Example: Preparation of Sasa albo-marginata extract

Dried leaves of the Sasa albo-marginata collected in TESHIO Mountains in Hokkaido Japan in September were introduced into a pressurized hot water extraction tank, treated at 125°C for 10 minutes in the tank, the hot water was cooled down to about 80°C by the action of a cooling water and then the resulting extract was separated from the moisture-containing solid content using a screw-press in such a manner that the moisture content of the latter was controlled to a level of about 50% by mass. Then the solid contents having a moisture content of about 50% by mass were introduced into an autoclave and heat-treated under pressure at 180°C for 10 minutes using saturated steam. The moisture-containing solid contents thus treated were again introduced into a pressurized hot water-extraction tank and treated at 110°C for 5 minutes to thus obtain an extract. The first and second extracts were combined together, filtered through a diatomaceous earth layer, the resulting filtrate was concentrated under reduced pressure till the solid content thereof was increased to 50% by mass and the concentrate thus prepared was subjected to a fluidized sterilization treatment at a temperature ranging from 110 to 130°C to give a Sasa albo-marginata extract.

The Sasa albo-marginata extract was inspected for the sulfur content and it was found to be 3850 µm/ml (7.7 mg per one gram of the solid content). Examples 1 to 4

The components listed in the following Table 2 were admixed together in amounts (% by mass) likewise specified in Table 2, introduced into a heating and mixing kettle equipped with a stirring blade and an emulsification apparatus and then mixed therein with stirring at 80°C for 2 hours to thus give an antipruritic cream composition according to the present invention. The added amounts of a Sasa albo-marginata extract having a solid content of 8% by mass (a product obtained by diluting the Sasa albo-marginata extract having a solid content of 50% by mass and prepared in Reference Example) were 12.5, 25, 37.5 and 75% by mass respectively (therefore, the contents of the extract as expressed in terms of the solid content thereof were 1, 2, 3 and 6% by mass; and sulfur contents of these samples were 7.7 mg, 15.4 mg, 23.1 mg and 46.2 mg per 100 g of the composition, respectively).

**Table 2**

| Component | Amount (% by mass) |
|---|---|
| Squalane | 5.0 |
| Olive oil | 6.0 |
| Lemon oil | 1.0 |
| Stearic acid | 4.0 |
| Glycerin monostearate | 0.8 |
| Carboxy vinyl polymer (CARBOPOL 940) | 0.2 |
| Glycerin monostearate (self-emulsifiable type) | 1.0 |
| Sodium N-acyl-L-glutamate | 0.2 |
| 1,3-Butylene glycol | 1.0 |
| Urea | 10.0 |
| Allantoin | 0.1 |
| Methyl p-oxy-benzoate | 0.1 |
| Propyl p-oxy-benzoate | 0.1 |
| Sasa albo-marginata extract (solid content 8% by mass) | Desired amount |
| Potassium hydroxide | 0.02 |
| Ion-exchange water | Balance |

### Comparative Example 1

The same procedures used in Example 1 were repeated except that the added amount of the Sasa albo-marginata extract having a solid content of 8% by mass (a product obtained by diluting the Sasa albo-marginata extract having a solid content of 50% by mass and prepared in Reference Example) was changed to 6.2% by mass (accordingly, the content of the Sasa albo-marginata extract (as expressed in terms of the solid content thereof) was 0.5% by mass and the sulfur content thereof was 3.8 mg per 100 g of the composition) to thus give an antipruritic cream composition of Comparative Example 1.

### Example 5

The same procedures used in Examples 1 to 4 were repeated using the components shown in the following Table 3 in the amounts likewise specified in Table 3 to thus give each corresponding antipruritic cream composition according to the present invention.

**Table 3**

| Component | Amount (% by mass) |
|---|---|
| Squalane | 1.0 |
| Olive oil | 4.0 |
| Orange oil | 1.0 |
| Octyl dodecyl myristate | 5.0 |
| Stearic acid | 4.0 |
| Cetanol | 2.0 |
| Polyethylene glycol distearate | 0.5 |
| Glycerin monostearate | 1.0 |
| Carboxy vinyl polymer (CARBOPOL 940) | 0.2 |
| Glycerin monostearate (self emulsifiable type) | 1.4 |
| Sodium N-acyl-L-glutamate | 0.2 |
| 1,3-Butylene glycol | 1.0 |
| Urea | 3.0 |
| Allantoin | 0.1 |
| Mixed fatty acid ester (NIKOGUARD DL) | 0.5 |
| Sasa albo-marginata extract (solid content: 8% by mass) | 75.0 |
| Potassium hydroxide | 0.05 |
| Ion-exchange water | Balance |

The composition has a sulfur content of 46.2 mg per 100 g thereof.

### Comparative Example 2

The Sasa albo-marginata extract, as such, having a solid content of 50% by mass and prepared in Reference Example was defined to be a sample of Comparative Example 2.

### Test Example 1 (Effect of alleviating symptoms accompanied by itching of patients suffering from atopy)

The antipruritic cream composition of Example 2 (the content of Sasa albo-marginata extract (solid content; in the following Table 4 "SC"): 2% by mass), the antipruritic cream composition of Example 4 (the content of Sasa albo-marginata extract (solid content): 6% by mass), the cream composition of Comparative Example 1 (the content of Sasa albo-marginata extract (solid content): 0.5% by mass) and the composition of Comparative Example 2 (the content of Sasa albo-marginata extract (solid content): 50% by mass) were applied to patients who developed the symptoms of atopy and the results shown in the following Table 4 were obtained.

**Table 4**

| Patient No. (age) | Symptoms | Method of Use and Period of use | Effect of alleviating or improving the symptoms |
|---|---|---|---|
| 1 (40*) | Wet, over entire body, especially, neck and hip | When feeling itchy, twice a day in the morning and evening, over about 180 days | Compositions having SC of 0.5, 2 and 50% did not have any effect. The composition having SC of 6% was considerably effective and almost 80% of the symptoms were restored. |
| 2 (18*) | Wet, over entire body | The compositions were applied once a day in the evening, over about 180 days | The compositions having SC of 0.5 and 50% did not have any effect. The symptoms were almost alleviated with the composition having SC of 2% and the latter was applied when feeling itchy. The composition having SC of 6% made the skin smart. |
| 3 (9) | Dry, over the entire body | Every day prior to going to bed or when feeling itchy, over about 180 days | The compositions having SC of 0.5 and 50% did not have any effect. The composition having SC of 2% was effective. The composition having SC of 6% made the skin smart. |
| 4( 41) | Dry, neck and hands | Every evening over about 150 days. | An effect was observed when the composition having SC of 2% was applied to the affected portions. |
| 5 (9*) | Dry, over the entire body | When feeling itchy, over about 150 days. | The composition having SC of 50% did not have any effect. The composition having SC of 2% was effective. |
| 6 (41) | Dry, over the entire body | Once everyday over about 150 days | The composition having SC of 50% did not have any effect. The sample having SC of 2% was effective. The sample having SC of 6% was highly effective. |
| 7 (28) | Dry, neck | Every evening over about 45 days | The composition having SC of 50% did not have any effect. The sample having SC of 2% was highly effective. |
| 8 (20*) | Dry, neck, armpit | Every evening over about 60 days | The compositions having SC of 0.5% and 50% did not have any effect. When applying one having SC of 2%, the symptoms were completely recovered and any composition has never been used recently. |
| 9 (80) | Dry, over the entire body, suffering from senile itch | Every evening over about 15 days | When applying one having SC of 2%, the symptoms were completely recovered and any composition has never been used recently. |
| 10 (83*) | Over the entire body, suffering from senile itch | When feeling itchy over about 90 days | The compositions having SC of 0.5% and 50% do not have any effect. One having SC of 2% permitted slight improvement of the symptoms. One having SC of 4% shows the optimum effect. |

| | | | |
|---|---|---|---|
| *: Male patient. | | | |

### Test Example 2 (Wound-Healing Effect on Patients Injured)

The antipruritic cream composition of Example 3 (the content of Sasa albo-marginata extract (solid content; in the following Table 5 "SC"): 3% by mass), the cream composition of Comparative Example 1 (the content of Sasa albo-marginata extract (solid content): 0.5% by mass) and the composition of Comparative Example 2 (the content of Sasa albo-marginata extract (solid content): 50% by mass) were applied to injured patients and the results shown in the following Table 5 were obtained. In most of patients tested, the symptoms of the injured portions were considerably improved or alleviated by the treatment over about 3 days.

**Table 5**

| Patient No. (age) | Symptoms | Effect of improving the symptoms |
|---|---|---|
| 11 (70) | Itching, a cut | The composition having SC of 50% made the skin smart and did not have any effect. One having SC of 3% showed a higher effect. |
| 12 (49) | A cut | One having SC of 3% was highly effective. One having SC of 0.5% did not have any effect. |
| 13 (28) | Sore by a chemical, a cut | The use of the composition having SC of 50% accompanied by pains. One having SC of 3% showed a higher effect. |
| 14 (72) | A cut, insect bites | The composition having SC of 3% showed a higher effect. One having SC of 0.5% did not have any effect. |
| 15 (67) | Scratch marks formed by a cat | The composition having SC of 3% showed a higher effect. One having SC of 0.5% did not have any effect. |
| 16 (35*) | Agnail, Scratch marks | The composition having SC of 3% showed a higher effect. One having SC of 0.5% did not have any effect. |
| 17 (66) | A cut | The composition having SC of 3% showed a higher effect. One having SC of 0.5% did not have any effect. |
| 18 (60*) | Injuries formed on lip and face upon falling down | The injuries were almost completely healed by treating them with the composition having SC of 3% over 3 days. |
| 19 (70*) | Cuts with a knife | The cuts were almost completely healed by treating them with the composition having SC of 3% over 2 days. |
| 20 (65*) | Cuts formed when falling down in a mountain | The cuts were almost completely healed by treating them with the composition having SC of 3% over 4 days. |
| 21 (3) | Abrasions | The cuts were almost completely healed by treating them with the composition having SC of 3% over 3 days. The use of one having SC of 50% was accompanied by pains. One having SC of 0.5% did not have any effect. |

| | | |
|---|---|---|
| *: Male patient. | | |

The foregoing results clearly indicate that the antipruritic composition of the present invention containing 1 to 10% by mass of the Sasa albo-marginata extract, as expressed in terms of the solid content thereof, shows a considerable antipruritic effect as compared with the Sasa albo-marginata extract per se having a solid content of 50% by mass, which is accompanied by pains upon application to the skin and is insufficient in the antipruritic effect, that the wound-healing-promoting composition of the present invention containing 1 to 10% by mass of the Sasa albo-marginata extract has a significant wound-healing-promoting effect, while the Sasa albo-marginata extract per se having a solid content of 50% by mass is accompanied by pains upon application to the skin and is insufficient in the wound-healing-promoting effect and that one having a solid content of 0.5% by mass is insufficient in the wound-healing-promoting effect.

## Claims

1. An antipruritic composition comprising Sasa albo-marginata water extract in an amount ranging from 1 to 10% by mass as expressed in terms of the solid content thereof.

2. The antipruritic composition of claim 1, wherein it comprises the sulfur components originated from Sasa albo-marginata in an amount ranging from 4 to 100 mg per 100 g of the composition as expressed in terms of the amount of sulfur.

3. The antipruritic composition of claim 1, wherein it comprises 2 to 8% by mass of the Sasa albo-marginata water extract as expressed in terms of the solid content thereof.

4. The antipruritic composition as set forth in any one of claims 1 to 3, wherein it is in the form of a cream.

5. Use of a composition according to claim 1 in the manufacture of a composition for promoting wound healing.

6. The use of claim 5 wherein the wound-healing-promoting composition comprises the sulfur components originated from Sasa albo-marginata in an amount ranging from 4 to 100 mg per 100 g of the composition as expressed in terms of the amount of sulfur.

7. The use of claim 5 wherein the wound-healing-promoting composition comprises 2 to 8% by mass of the Sasa albo-marginata water extract as expressed in terms of the solid content thereof.

8. The use as set forth in any one of claims 5 to 7, wherein the wound-healing-promoting composition is in the form of a cream.

## Patentansprüche

1. Antipruriginöse Zusammensetzung, umfassend Sasa albomarginata-Wasserextrakt in einer Menge im Bereich von 1 bis 10 Massen-%, wie in Form des Feststoffgehalts davon ausgedrückt.

2. Antipruriginöse Zusammensetzung nach Anspruch 1, wobei diese die von Sasa albomarginata stammenden Schwefelkomponenten in einer Menge im Bereich von 4 bis 100 mg pro 100 g der Zusammensetzung, wie in Form der Menge an Schwefel ausgedrückt, umfasst.

3. Antipruriginöse Zusammensetzung nach Anspruch 1, wobei diese 2 bis 8 Massen-% des Sasa albomarginata-Wasserextrakts, wie in Form des Feststoffgehalts davon ausgedrückt, umfasst.

4. Antipruriginöse Zusammensetzung nach einem der Ansprüche 1 bis 3, wobei diese in der Form einer Creme vorliegt.

5. Verwendung einer Zusammensetzung nach Anspruch 1 in der Herstellung einer Zusammensetzung zur Förderung der Wundheilung.

6. Verwendung nach Anspruch 5, wobei die wundheilungsfördernde Zusammensetzung die von Sasa albomarginata stammenden Schwefelkomponenten in einer Menge im Bereich von 4 bis 100 mg pro 100 g der Zusammensetzung, wie in Form der Menge an Schwefel ausgedrückt, umfasst.

7. Verwendung nach Anspruch 5, wobei die wundheilungsfördernde Zusammensetzung 2 bis 8 Massen-% des Sasa albomarginata-Wasserextrakts, wie in Form des Feststoffgehalts davon ausgedrückt, umfasst.

8. Verwendung nach einem der Ansprüche 5 bis 7, wobei die wundheilungsfördernde Zusammensetzung in der Form einer Creme vorliegt.

## Revendications

1. Composition antiprurigineuse comprenant un extrait aqueux de Sasa albo-marginata en une quantité dans la plage de 1 à 10 % en masse, ceci exprimé en termes de teneur en matière sèche de celui-ci.

2. Composition antiprurigineuse selon la revendication 1, laquelle comprend les composants soufrés issus de Sasa albo-marginata en une quantité dans la plage de 4 à 100 mg pour 100 g de la composition, ceci exprimé en termes de quantité de soufre.

3. Composition antiprurigineuse selon la revendication 1, laquelle comprend 2 à 8 % en masse de l'extrait aqueux de Sasa albo-marginata, ceci exprimé en termes de teneur en matière sèche de celui-ci.

4. Composition antiprurigineuse selon l'une quelconque des revendications 1 à 3, laquelle est sous forme de crème.

5. Utilisation d'une composition selon la revendication 1 dans la fabrication d'une composition destinée à favoriser la cicatrisation des blessures.

6. Utilisation selon la revendication 5, dans laquelle la composition favorisant la cicatrisation des blessures comprend les composants soufrés issus de Sasa albo-marginata en une quantité dans la plage de 4 à 100 mg pour 100 g de la composition, ceci exprimé en termes de quantité de soufre.

7. Utilisation selon la revendication 5, dans laquelle la composition favorisant la cicatrisation des blessures comprend 2 à 8 % en masse de l'extrait aqueux de Sasa albo-marginata, ceci exprimé en termes de teneur en matière sèche de celui-ci.

8. Utilisation selon l'une quelconque des revendications 5 à 7, dans laquelle la composition favorisant la cicatrisation des blessures est sous forme de crème.
